# EUROPEAN PATENT APPLICATION

(11) **EP 3 427 666 A1**
(43) Date of publication of application: **16.01.2019**
(21) Application number: 17180765.4
(22) Date of filing: 11.07.2017
(51) Int. Cl.: A61B 6/03, A61B 6/00, G01R 33/48

(54) **COMBINED IMAGING SYSTEM WITH A MAGNETIC RESONANCE APPARATUS AND AN X-RAY APPARATUS**

(71) Applicant: Siemens Healthcare GmbH, 91052 Erlangen (DE)
(72) Inventor: Ertel, Dirk, 91301 Forchheim (DE); Kyriakou, Yiannis, 91080 Spardorf (DE)

(57) **Abstract**

The invention relates to an imaging system (1), which comprises an imaging magnetic resonance apparatus (2) with a magnetic resonance capturing area (3), which is formed for creating a magnetic resonance tomography of an object (4) to be imaged that is disposed in the magnetic resonance capturing area (3); and comprises an imaging X-ray apparatus (6) with an X-ray capturing area (7), which is formed for creating an X-ray image of the object (4) to be imaged that is disposed in the X-ray capturing area (7); wherein the imaging system (1) has a longitudinal axis (L) and the magnetic resonance capturing area (3) and the X-ray capturing area (7) are disposed overlapping at least in certain areas in a projection to a plane perpendicular to the longitudinal axis (L), and the X-ray apparatus (6) comprises at least two X-ray sources (8a-8g, 8u-8z) to overcome the known restrictions in the configuration of the X-ray capturing area in combined imaging systems.

## Description

The invention relates to an imaging system, which comprises an imaging magnetic resonance apparatus with a magnetic resonance capturing area, which is formed for creating a magnetic resonance tomography of an object to be imaged that is disposed in the magnetic resonance capturing area, and comprises an imaging X-ray apparatus with an X-ray capturing area, which is formed for creating a X-ray image of the object to be imaged that is disposed in the X-ray capturing area, wherein the imaging system has a longitudinal axis and the magnetic resonance capturing area and the X-ray capturing area are disposed overlapping at least in certain areas in a projection to a plane perpendicular to a longitudinal axis in the plane. The invention also relates to a method for operating such an imaging system.

Typically, a combined imaging system, for example an angiographic magnetic resonance apparatus, includes an X-ray apparatus with an X-ray tube as an X-ray source and a detector or a detector device in addition to the magnetic resonance apparatus. However, with the currently known X-ray technology, it is difficult to bring a high-power X-ray tube, thus a high-power X-ray source, into the magnetic field of the magnetic resonance apparatus. Here, the shape of the magnetic field generally only allows arrangement of the X-ray source in specific positions. At the same time, the X-ray source is restricted in its ray geometry, whereby the X-ray capturing area, thus the measurement field of the X-ray apparatus and correspondingly the illumination of the object, is restricted.

Thus, there is the object to provide a combined imaging system with magnetic resonance apparatus and X-ray apparatus, which overcomes the described restrictions in the configuration of the X-ray capturing area.

This object is solved by the subject matters of the independent claims. Advantageous embodiments are apparent from the dependent claims, the description and the figures.

The invention relates to an imaging system, which can also be referred to as combined imaging system, which comprises an imaging magnetic resonance apparatus with a magnetic resonance capturing area, which is formed for creating a magnetic resonance tomography of an object to be imaged that is disposed or placed in the magnetic resonance capturing area, an area infiltrated by a magnetic field of the magnetic resonance apparatus in the operation of the magnetic resonance apparatus. The imaging system also comprises an imaging X-ray apparatus with an X-ray capturing area, an area irradiated by X-rays or X-ray light in the operation of the X-ray apparatus, which is formed for creating an X-ray image of the object to be imaged that is disposed or placed in the X-ray capturing area. Therein, the magnetic resonance capturing area and the X-ray capturing area can overlap at least in certain areas, thus in certain areas, largely or completely. Here, a portion of 80 percent or more, in particular 90 percent or more, preferably 95 percent or more can be understood by largely or in large part. The imaging system can in particular be an imaging angiographic system.

Therein, the imaging system has a longitudinal axis, wherein the magnetic resonance capturing area and the X-ray capturing area are disposed overlapping at least in certain areas (thus in certain areas, largely or completely) in an orthogonal projection to a plane perpendicular to the longitudinal axis in the plane such that the object to be imaged can be pushed or slid into both capturing areas, thus the magnetic resonance capturing area and the X-ray capturing area, at the same time along the longitudinal axis.

Therein, the X-ray apparatus comprises at least two X-ray sources, the respective radiation cones of which form the X-ray capturing area for X-ray light or X-rays emitted by them in the operation of the X-ray sources. In particular, the X-ray apparatus also comprises at least one detector device with a corresponding detector surface, which can be irradiated with X-ray light by the X-ray sources, in addition to the X-ray sources. Therein, the detector device is preferably disposed diametrically opposing the X-ray sources on a lateral surface of a cylinder having the longitudinal axis as the central axis. Therein, the longitudinal axis in particular extends through the X-ray capturing area and/or the magnetic resonance capturing area. Therein, it can be provided that the X-ray sources are disposed rotatable around the longitudinal axis with the detector device. Therein, the longitudinal axis can be a longitudinal axis of an annular tunnel, a so-called gantry, in particular also a central axis. The annular tunnel can be associated with the magnetic resonance apparatus. Therein, the X-ray source and the detector device can be disposed at or attached to the annular tunnel. The X-ray apparatus can in particular be an X-ray apparatus suitable for positron emission tomography and/or single photon emission tomography and/or computer tomography.

Here, the use of multiple X-ray sources opens quite a series of specific embodiments explained in the following, which all solve the described problems. Namely, by the use of a single X-ray source or a single X-ray tube, for example if the X-ray source is disposed or arranged at an annular tunnel without tilting possibility, inclined recordings are not possible. Imaging outside of the annular tunnel itself, which is possibly required in more complex cases, either is not realizable with a single X-ray source, since in such cases for imaging and navigation within the vessel configuration, an image of a body part disposed outside of the annular tunnel as the object to be imaged, for example of arteries or veins in the hip area, typically has to be recorded at the same time and simultaneously a further image in a main capturing area or main measurement field, in which for example a heart has to be imaged with the magnetic resonance apparatus and with the X-ray apparatus at the same time.

Thus, the measurement field and the illumination of the object can also be configured substantially more flexibly. Therein, the requirements to the used X-ray source decrease with regard to the required power, which in turn increases the compatibility with the magnetic field of the magnetic resonance. By the use of multiple X-ray sources, thus, the matrix arrangement explained in the following for example becomes possible, and an individual control of individual X-ray sources also allows local and energetic, in particular spectral, variation in imaging. Compared to known arrangements with a single X-ray source, existing performance deficiencies are thereby compensated for. In particular, the proposed approach is also compatible for use with new X-ray technologies. Especially the multi-spectral imaging is allowed by the use of multiple X-ray sources described below, which also allows dose optimization and/or image optimization such that the object to be imaged can for example be decomposed in different materials in the picture, thus a material decomposition can be performed or automatic exposure can be used.

Finally, different areas in the X-ray capturing area can also be irradiated by the two X-ray sources such that a virtual collimator function can also be realized via the individual control explained below. Finally, by the use of multiple X-ray sources, the X-ray capturing area can also be extended, for example to an area, which is outside of the annular tunnel of the imaging system or outside of the magnetic resonance capturing area, such that specific medical interventions simpler proceed. Finally, by the use of multiple X-ray sources, scattered radiation can also be reduced in corresponding operation, for example by sequentially irradiating and reading out the detector device.

The radiation cones of the X-ray sources can preferably adjoin to each other or transition into each other or overlap in order to thus form a contiguous X-ray capturing area. Therein, the X-ray capturing area in particular completely covers the magnetic resonance capturing area or is completely in it.

Therein, the X-ray capturing area can be a contiguous X-ray capturing area or include multiple X-ray capturing sub-areas, which do not overlap and/or do not adjoin to each other. The object to be imaged can also to be imaged only respectively in certain areas by the magnetic resonance apparatus or X-ray apparatus, that is for example be disposed in a first object area in the magnetic resonance capturing area and be disposed in a second different object area in the X-ray capturing area.

In an advantageous embodiment, it is provided that the X-ray apparatus comprises at least four X-ray sources, which are disposed in the form of a matrix. Therein, the four X-ray sources can thus be disposed in at least one row and at least one line, wherein rows and lines are here preferably perpendicular or perpendicular to each other with a deviation of less than 15, preferably less than 5 degrees. In particular, the matrix can be a 1-by-4 or 2-by-2 matrix with four X-ray sources. Thus, the X-ray sources can in particular be disposed in the form of a square matrix, for example in the form of a 2-by-2 or 4-by-4 of 8-by-8 matrix. In an alternative preferred embodiment, the X-ray sources are therein disposed in a one-dimensional matrix, thus a 1-by-4, 1-by-8, generally 1-by-x matrix. Here, the row(s) or the line(s) can in particular extend parallel to the longitudinal axis or extend parallel to each other with a deviation of less than 15 degrees, preferably less than 5 degrees.

Especially by the matrix form, a plurality of useful geometries for the X-ray capturing area arises for the corresponding associated radiation cones such that it can be particularly flexibly configured. Therein, a greater number of X-ray sources is basically advantageous since the individual X-ray sources are then correspondingly equipped with less power and smaller cones than the X-ray sources with a lower number of X-ray sources such that more power is achieved per solid angle even by a weaker X-ray source than if the respective X-ray sources are to illuminate a larger radiation cone. Here, more powerful X-ray sources can be correspondingly omitted by a greater number of X-ray sources since in particular a power gain is achieved by the plurality of X-ray sources. Therein, the individual X-ray sources can for example sequentially irradiate or illuminate the detector device or a detector surface of the detector such that considerably less scattered radiation arises at the detector device. This for example results in the fact that a scattered ray raster also becomes expendable and thus the overall construction becomes simpler and more flexible.

In a further advantageous embodiment, it is provided that the X-ray sources are at least partially disposed in a first row, which extends in a plane perpendicular to the longitudinal axis, and/or are at least partially, thus partially, largely or completely, disposed in a second row (which can also be referred to as line), which extends parallel to the longitudinal axis.

This orientation of the X-ray sources or of the X-ray source matrix has proven particularly advantageous since particularly useful geometries of the X-ray capturing area can be achieved by it in flexible manner.

In a further advantageous embodiment, it is provided that the X-ray sources each comprise a stationary anode tube and/or have a power of less than three kilowatts, in particular less than two kilowatts. Thus, the X-ray apparatus comprises multiple X-ray sources, which offer short distances for the acceleration of the electrons and thus are preferably not dependent on rotating anodes. Thereby, increased compatibility with the magnetic resonance apparatus is achieved and moreover the already described increased flexibility in the configuration of the geometry of the X-ray capturing area is achieved in synergetic manner. Therein, the stationary anode tubes can have one or more foci and/or also include one or more carbon nanotubes.

In a further advantageous embodiment, it is provided that at least a subset of the X-ray sources, in particular all of the X-ray sources, is individually controllable. In particular, the individually controllable X-ray sources and thereby their radiation cones are pivotable parallel to the longitudinal axis, thus in a plane extending through the longitudinal axis, and/or in the plane perpendicular to the longitudinal axis. Alternatively or additionally, the individually controllable X-ray sources and thereby their radiation cones can be individually combinable. The subset of the individually controllable X-ray sources can for example exclusively include the X-ray sources disposed at the edge of the matrix. Alternatively or additionally, all of the X-ray sources can also be individually controllable, for example can be individually combinable, and only a subsidiary subset, for example the X-ray sources disposed at the edge of the matrix, can be pivotable parallel to the longitudinal axis and/or in the plane perpendicular to the longitudinal axis.

By the capability of pivoting, here, it can be switched back and forth between different operating modes explained in the following, in which the corresponding radiation cones can more or less overlap depending on the clinical issue. In addition, by the individual control of the X-ray sources, a virtual collimation of the ray can be achieved in a collimation operating mode such that more complicated collimator arrangements are not required and the overall construction is further simplified and flexibilized. In this collimation operating mode, only the X-ray sources are activated or switched, which are required to radiograph a spatial area preset by the imaging system or a user, wherein here a volume situated around the preset spatial area is not radiographed or irradiated corresponding to a collimator function.

However, a collimator can additionally also be provided, which can function in the form of a shutter device such that a small section from the radiation cone of the respectively active X-ray source can be utilized for radiographing the object to be imaged. Therein, in a further operating mode, a series of smaller radiation cone sections can for example be serially used for radiographing, such that overall a larger field of vision (FOV) is radiographed by the series. Thereby, even smaller spatial areas can be irradiated and a respective shape for the volume to be radiographed can be correspondingly individually flexibilized preset. Therein, the sum of all of the radiation cones can correspond to the entire X-ray capturing area.

Thus, the advantage arises that different radiation fields or different X-ray capturing areas, in particular X-ray capturing areas with different geometries, can be generated. The corresponding signals of the detector device associated with the individual X-ray sources can subsequently be converted to a common image. This can for example occur by suitable multiplexing. At the same time, for the multi-spectral imaging with X-ray lights of different spectra explained in the following, each signal of the detector device can also be individually evaluated before multiplexing if required. Therein, the X-ray sources can in particular be controllable such that each X-ray source illuminates or irradiates a dedicated area on the detector surface of the detector. Similarly, the same area on the detector surface can also be illuminated by two sources or more sources if one wants to perform for example a two- or multi-spectral analysis for this area or an associated spatial area in the capturing area. Thus, the spectrum can for example be optimized and/or kept variable depending on a clinical issue within a sum cone, thus a superposition of at least two radiation cones.

By the capability of individual control, a computer tomography-like imaging can be allowed by the X-ray apparatus. By suitable switching of the X-ray sources and corresponding readout of the detector device, here, less scattered radiation is generated as mentioned above. In particular, scattered radiation contributions can also be avoided in an interleaved operating mode, in which only areas of the detector surface are irradiated in a preset time interval, which are spatially spaced from each other, and thus the image quality can be improved.

In a further advantageous embodiment, it is provided that at least a subset of X-ray sources is disposed oriented or orientable, for example pivotable, such that respective radiation cones of the X-ray sources at least partially overlap, preferably largely overlap, on a detector device, that is on an associated detector surface of the detector device, in the operation thereof. For example, the subset can each include a pair of X-ray sources, but also respectively three or more X-ray sources. The X-ray sources of such a subset then commonly irradiate an area of the detector surface of the detector device. In particular, the subset can include all of the X-ray sources of the X-ray apparatus, that is for example all of the X-ray sources can irradiate one and the same detector surface or the partial surface of the detector surface irradiated by the respective X-ray sources can largely coincide with the partial surface irradiated by the corresponding other X-ray sources of the subset, thus in particular all of the X-ray sources.

Therein, the radiation cones can in particular overlap only in an overlap operating mode of the X-ray apparatus in the described manner if the associated X-ray sources are pivotable. In this case, the X-ray apparatus can therefore be switched back and forth between a normal operating mode, in which the radiation cones do not or only insignificantly overlap, thus by less than 20 percent, less than 10 percent or less than 5 percent, and the overlap operating mode, which correspondingly is associated with pivoting of the X-ray sources. Here, oriented can be understood in terms of aligned.

By pivoting, the tubes can also be oriented such that inclined recordings, so-called cranial-caudal angulations, can be performed or simulated. Thereto, the X-ray sources at the edge of the matrix can in particular be oriented such that a (virtual) inclined picture can arise.

This has the advantage that in control (similar to the known inverse geometry), a detector device with a large detector surface can be omitted, and only a small detector surface is required. By the corresponding individual cone illumination, thus, serial illumination of the respective radiation cones with X-ray light, a desired spatial area can nevertheless be completely illuminated. This saves a detector device with a large detector surface one the one hand and allows using specialized high-resolution detector surfaces on the other hand, which are not yet available with larger dimensions. At the same time, space is also saved.

Therein, it can be provided in an advantageous embodiment that the X-ray lights or X-rays emitted by the X-ray sources associated with the respective subset have different spectra, that is qualitatively and/or quantitatively different from each other.

This has the advantage that the multi-spectral imaging already described above is possible, which for example can be used for a so-called material disassembly or material decomposition in a generated image. For example, this can occur by suitable multiplexing.

In a further advantageous embodiment, it is provided that at least one, preferably two or more X-ray sources are oriented or orientable, for example pivotable, such that X-ray light of the radiation cone of the X-ray source irradiates an associated further detector device partially or completely disposed outside of the magnetic resonance capturing area in the longitudinal axis in the operation thereof, in particular at least partially, preferably largely or completely irradiates a respective detector surface of the further detector device. In particular, an X-ray source disposed at the edge of the matrix or X-ray sources disposed at a part of the edge or circumferentially at the entire edge of the matrix can be thus oriented or orientable.

Therein, the X-ray light can in particular irradiate the detector surface disposed outside of the magnetic resonance capturing area only in an extension operating mode of the X-ray apparatus if the associated X-ray source is pivotable. In this case, the X-ray apparatus can thus be operated in the extension operating mode, in which the detector surface disposed outside of the magnetic resonance capturing area is irradiated, or in a normal operating mode, in which the detector surface at least partially or largely disposed within the magnetic resonance capturing area is then for example irradiated. Therein, the further detector device or the associated detector surfaces can thus be mounted on a moving unit outside of the annular tunnel to extend the longitudinal X-ray measurement field, the X-ray capturing area, in the direction of the longitudinal axis.

This has the advantage that the geometry of the available X-ray capturing area can be fast adapted to the respective situation or the respective intervention with little effort in flexible and adequate manner.

Therein, it is provided in an advantageous embodiment that the further detector device comprises at least one, preferably two detector surfaces, which are configured displaceable movable, that is, slidable, in the longitudinal axis, in particular disposed in displaceable manner at the annular tunnel in intended use. Therein, the respective detector surface can for example remain oriented parallel to the longitudinal axis upon displacing the longitudinal axis or be formed such that it is only displaceable parallel to the longitudinal axis. Thus, the detector surface can be mounted on a moving unit. Therein, the one or more detector surfaces in particular supplement the detector surface of the above mentioned (first) detector device, which is fixedly mounted on the annular tunnel. If two further detector devices or detector surfaces are present, thus, they can be disposed on different sides of the magnetic resonance capturing area in the direction of the longitudinal axis starting from the magnetic resonance capturing area and/or be displaceable in different directions.

This has the advantage that a particularly great flexibility is achieved and the initially mentioned problems of different areas of an object to be observed during a medical intervention, for example a hip and a head or a heart of a patient, are avoided.

Therein, it is provided in a particularly advantageous embodiment that the imaging system includes or is a medical imaging system. Here, the mentioned flexibility is particularly advantageous and desirable.

The invention also relates to a method for operating an imaging system, which comprises an imaging magnetic resonance apparatus with a magnetic resonance capturing area, which is formed for creating a magnetic resonance tomography of an object to be imaged disposed in the magnetic resonance capturing area, and comprises an imaging X-ray apparatus with an X-ray capturing area, which is formed for creating an X-ray image of the object to be imaged disposed in the X-ray capturing area. Therein, the imaging system has a longitudinal axis and the magnetic resonance capturing area and the X-ray capturing area are disposed overlapping at least in certain areas in a projection to the plane perpendicular to the longitudinal axis. Therein, an object located in the X-ray capturing area is radiographed or irradiated by X-ray light of at least two different X-ray sources of the X-ray apparatus. Advantages and advantageous embodiments of the method correspond to advantages and advantageous embodiments of the described imaging system.

The features and feature combinations mentioned above in the description as well as the features and feature combinations mentioned below in the description of figures and/or shown in the figures alone are usable not only in the respectively specified combination, but also in other combinations without departing from the scope of the invention. Thus, implementations are also to be considered as encompassed and disclosed by the invention, which are not explicitly shown in the figures and explained, but arise from and can be generated by separated feature combinations from the explained implementations. Implementations and feature combinations are also to be considered as disclosed, which thus do not have all of the features of an originally formulated independent claim. Moreover, implementations and feature combinations are to be considered as disclosed, in particular by the implementations set out above, which extend beyond or deviate from the feature combinations set out in the relations of the claims.

Below, embodiments of the invention are explained in more detail based on schematic drawings. There show:
- FIG 1: a schematic longitudinal view of an exemplary embodiment of an imaging system;
- FIG 2: a schematic transverse view of the embodiment of FIG 1;
- FIG 3: a schematic transverse view of a further exemplary embodiment; and
- FIG 4: a schematic longitudinal view of another exemplary embodiment of an imaging system.

In the figures, identical or functionally identical elements are provided with the same reference characters.

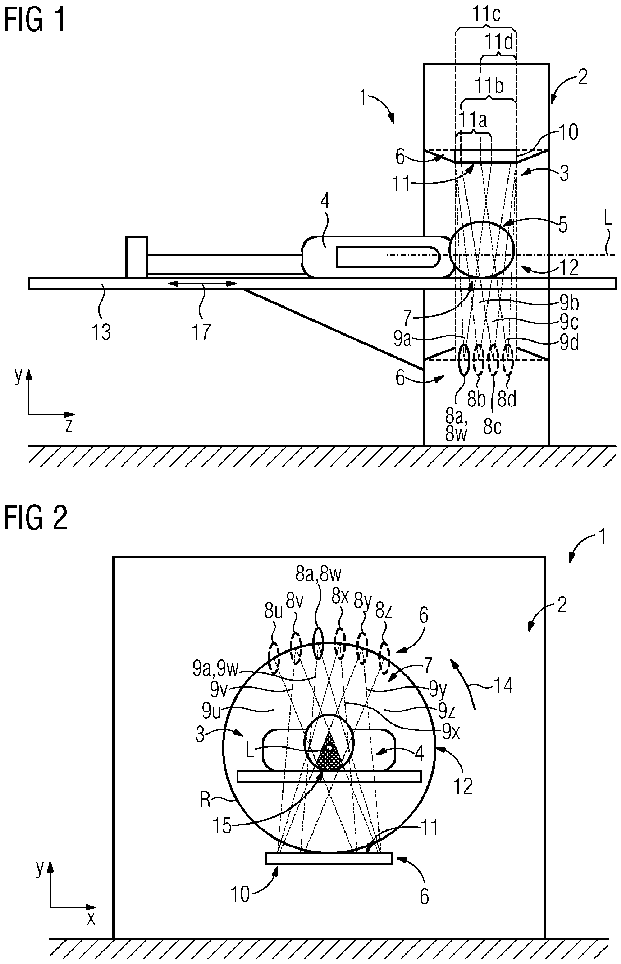

In FIG 1, an exemplary embodiment of an imaging system is illustrated in a longitudinal view. Therein, the imaging system 1 comprises an imaging magnetic resonance apparatus 2 with a magnetic resonance capturing area 3. It is formed for creating a magnetic resonance tomography of an object 4 to be imaged disposed in the magnetic resonance capturing area 3. Presently, a patient as the object 4 to be imaged is partially disposed in the magnetic resonance capturing area 3, namely with his head 5.

The imaging system 1 also comprises an imaging X-ray apparatus 6 with an X-ray capturing area 7. Therein, the imaging X-ray apparatus 6 is formed for creating an X-ray image of the object 4 to be imaged disposed in the X-ray capturing area 7. Therein, the imaging system has a longitudinal axis L here extending in z-direction and the magnetic resonance capturing area 3 and the X-ray capturing area 7 overlap at least in certain areas in a projection to a plane perpendicular to the longitudinal axis L, as it is even more clearly recognizable in FIG 2. Presently, the capturing areas also overlap in the represented y-z plane, in which the longitudinal axis L is situated.

Therein, the X-ray apparatus 6 comprises at least two X-ray sources, presently four X-ray sources 8a to 8d. Therein, the correspondingly associated radiation cones 9a to 9d, which are preset by the X-rays, the X-ray light, exiting the X-ray sources 8a to 8d in the operation of the X-ray apparatus 6, presently form the X-ray capturing area 7. The X-ray apparatus 6 also comprises a detector device 10 with a detector surface 11. The detector surface 11 is irradiated in different detector surface areas 11a to 11d by the respective X-ray sources 8a to 8d. In the shown example, the detector device 10 is disposed diametrically opposing the X-ray sources 8a to 8d on a circle around the longitudinal axis L such that a patient as the object 4 shifted into the annular tunnel 12 of the magnetic resonance apparatus 2 in the longitudinal axis L can be shifted into both capturing areas 3, 7. For this purpose, the imaging system 1 presently comprises a table 13 displaceable along the longitudinal axis L. The table 13 is displaceable in positive and negative z-direction as indicated by the double arrow 17.

The shown X-ray sources 8a to 8d of the X-ray apparatus 6 are presently disposed in a row parallel to the longitudinal axis L. Therein, the X-ray source 8a of the shown row presently corresponds to the X-ray source 8w of the line of X-ray sources 8u to 8z shown in FIG 2 of a matrix of here 4x6 X-ray sources. Correspondingly, the X-ray sources 8a to 8d irradiate respective partial surfaces 11a to 11d of the detector surface 11. In that the X-ray sources 8a to 8d presently can be individually controlled, thus, individual areas of the head 5 can for example be specifically radiographed and thus imaged by the X-ray apparatus 5, such that a collimator function becomes at least partially unnecessary. Moreover, in the different radiation cones 9a to 9d, different X-rays, that is X-rays with different spectra, can thus also be used to thus for example be able to perform material decomposition.

In FIG 2, the imaging system of FIG 1 is illustrated in a schematic transverse view perpendicular to the longitudinal axis L. Therein, it becomes clear that the X-ray apparatus 6 comprises further X-ray sources 8u to 8z, which are presently disposed in a row forming a line of the matrix of X-ray sources, which extends in the x-y plane, thus is disposed perpendicular to the longitudinal axis L. Therein, the further X-ray sources 8u to 8z can be disposed diametrically opposing the associated detector device 10 on a radius R around the longitudinal axis L. Overall, the present imaging system 1 therefore comprises four rows of respectively six further X-ray sources 8u to 8z. Thus, the X-ray source 8a presently corresponds to the X-ray source 8w and the X-ray sources 8a to 8d, 8u to 8z, overall form a matrix of presently 4 by 6 X-ray sources with further X-ray sources not represented.

Here, the corresponding radiation cones 9a to 9d and 9u to 9z, respectively, overlap in certain areas such that a virtual collimation of the X-radiation can be achieved by individual control of the X-ray sources 8a to 8d and 8u to 8z on the one hand, if multiple X-ray sources illuminate a same spatial area within the X-ray capturing area 7, in which the object 4 is disposed, such as for example presently the X-ray sources 8v and 8y with their radiation cones 9v and 9y illuminate the spatial area 15, a multi-spectral analysis or multi-spectral imaging with for example a corresponding material decomposition is also possible on the other hand via different spectra of the two X-ray sources 8v, 8y.

Presently, the X-ray apparatus 6 is disposed rotatable around the longitudinal axis L as indicated by the arrow 14. In rotating, the relative position of the detector 10 to the X-ray sources 8u to 8z and 8a to 8d, respectively, (FIG 1) does not change.

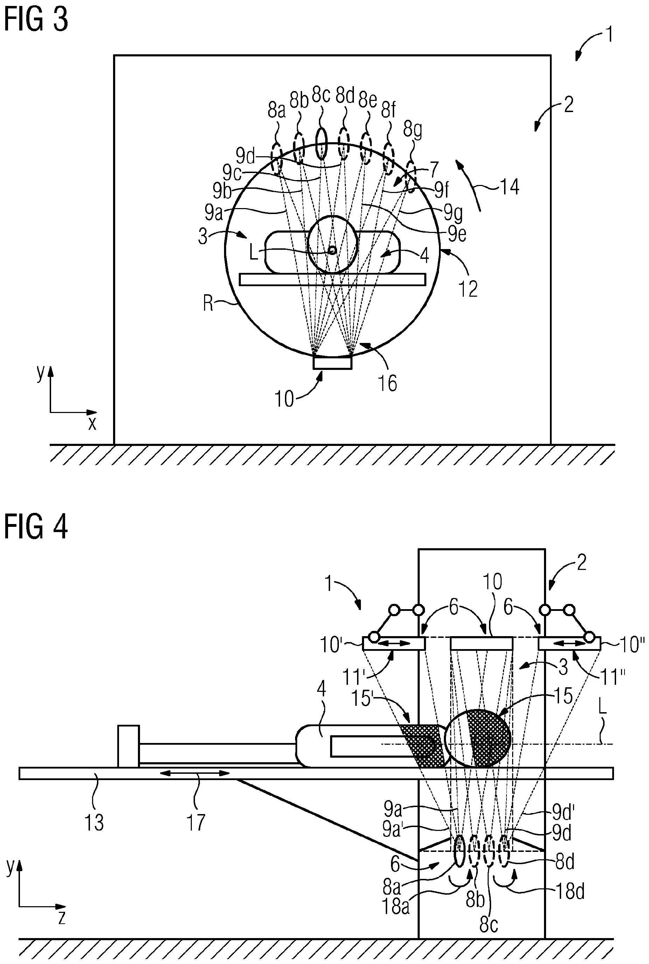

In FIG 3, a further exemplary embodiment of an imaging system is now illustrated in a schematic transverse view. Presently, seven X-ray sources 8a to 8d, which are here disposed opposing the detector 10 on the radius R around the longitudinal axis L, are here present. Therein, the detector 10 is presently substantially smaller than in the example shown in FIG 2 such that the capturing area 7, which is composed of the radiation cones 9a to 9g, has less than half of the overall extension in x-direction, presently a third of the overall extension in the x-direction perpendicular to the longitudinal axis L in an area 16 close to the detector. Therein, the overall extension in x-direction is presently determined by the maximum distance of the radiation cone edge areas facing away from each other in an area 19 close to the source. This is referred to as so-called inverse geometry since the capturing area of an X-ray apparatus is generally formed by a radiation cone in the X-ray imaging, which increases in its width or in its extension starting from the X-ray source towards the detector.

Presently, via a serial individual control or activation of the corresponding X-ray sources 8a to 8g, the object 4 can nevertheless be imaged in the entire capturing area 7 thanks to corresponding multiplexing with the serial control of the X-ray tube and the detector. The scattered radiation reduced therein as well as the possibility of using highly specific high-resolution detectors, which are only available in low constructional sizes, here also results in flexibilization of the imaging system 1 for different medical scenarios of employment.

In FIG 4, a further example of an imaging system is now illustrated. Here, the X-ray sources 8a to 8d are disposed parallel to the longitudinal axis L in a row corresponding to the example illustrated in FIG 1. Therein, the two X-ray sources 8a, 8d at the edge of the row, thus, at the edge of the matrix in the general case, are pivotably disposed, which is symbolized by the two arrows 18a, 18d. Correspondingly, the radiation cones 9a, 9d are pivotable into the positions indicated by 9a' and 9d'. Presently, at least one, presently two further detector devices 10', 10" with respective additional detector surfaces 11', 11" of the detector device 10 displaceable in z-direction parallel to the longitudinal axis L are correspondingly present in addition to a detector surface 11 of the detector device 10 fixedly disposed at the annular tunnel 12. Thereby, an area 15' located outside of the magnetic resonance capturing area 3 can be radiographed, namely additionally, in particular parallel to an area 15 of the object 4 located both in the magnetic resonance capturing area 3 and in the X-ray capturing area 7.

## Claims

1. Imaging system (1), which comprises
- an imaging magnetic resonance apparatus (2) with a magnetic resonance capturing area (3), which is formed for creating a magnetic resonance tomography of an object (4) to be imaged that is disposed in the magnetic resonance capturing area (3); and
- an imaging X-ray apparatus (6) with an X-ray capturing area (7), which is formed for creating an X-ray image of the object (4) to be imaged that is disposed in the X-ray capturing area (7);
wherein the imaging system (1) has a longitudinal axis (L) and the magnetic resonance capturing area (3) and the X-ray capturing area (7) are disposed overlapping at least in certain areas in a projection to a plane perpendicular to the longitudinal axis (L),
**characterized in that**
the X-ray apparatus (6) comprises at least two X-ray sources (8a-8g, 8u-8z).

2. Imaging system (1) according to claim 1, **characterized in that** the X-ray apparatus (6) comprises at least four X-ray sources (8a-8g, 8u-8z), which are disposed in the form of a matrix, in particular in the form of a square matrix.

3. Imaging system (1) according to any one of the preceding claims, **characterized in that** the X-ray sources (8a-8g, 8u-8z) are at least partially disposed in a row, which extends in a plane perpendicular to the longitudinal axis (L) and/or are at least partially disposed in a row, which extends parallel to the longitudinal axis (L).

4. Imaging system (1) according to any one of the preceding claims, **characterized in that** the X-ray sources (8a-8g, 8u-8z) each comprise a stationary anode tube and/or have a power of less than 3 kilowatts, in particular less than 2 kilowatts.

5. Imaging system (1) according to any one of the preceding claims, **characterized in that** at least a subset of the X-ray sources (8a-8g, 8u-8z), in particular all of the X-ray sources (8a-8g, 8u-8z), are individually controllable, in particular the individually controllable X-ray sources (8a-8g, 8u-8z) are pivotable parallel to the longitudinal axis (L) and/or in the plane perpendicular to the longitudinal axis (L) and/or the individually controllable X-ray sources (8a-8g, 8u-8z) can be individually collimated.

6. Imaging system (1) according to any one of the preceding claims, **characterized in that** at least a subset of X-ray sources (8a-8g, 8u-8z) is oriented such that respective radiation cones (9a-9g, 9u-9z) of the X-ray sources (8a-8g, 8u-8z) at least partially overlap, preferably largely overlap, on a detector device (6) in the operation thereof, in particular the subset includes all of the X-ray sources (8a-8g, 8u-8z) of the X-ray apparatus (6).

7. Imaging system (1) according to claim 6, **characterized in that** the X-ray lights emitted by the X-ray sources (8a-8g, 8u-8z) associated with the respective subset have different spectra.

8. Imaging system (1) according to any one of the preceding claims, **characterized in that** at least one X-ray source (8a-8g, 8u-8z) is oriented such that X-ray light of the X-ray source (8a-8g, 8u-8z) at least partially, preferably largely, irradiates an associated further detector device (10', 10") disposed partially or completely outside of the magnetic resonance capturing area (3) in the longitudinal axis (L) in the operation thereof.

9. Imaging system (1) according to claim 8 and 5, **characterized in that** the further detector device (10', 10") comprises at least one detector surface (11', 11"), which is configured displaceable movable in the longitudinal axis (L).

10. Imaging system (1) according to any one of the preceding claims, **characterized in that** the imaging system (1) includes a medical imaging system.

11. Method for operating an imaging system (1), which
- comprises an imaging magnetic resonance apparatus (2) with a magnetic resonance capturing area (3), which is formed for creating a magnetic resonance tomography of an object (4) to be imaged that is disposed in the magnetic resonance capturing area (3); and
- comprises an imaging X-ray apparatus (6) with an X-ray capturing area (7), which is formed for creating an X-ray image of the object (4) to be imaged that is disposed in the X-ray capturing area (7);
wherein the imaging system (1) has a longitudinal axis (L), as well as the magnetic resonance capturing area (3) and the X-ray capturing area (7) are disposed overlapping at least in certain areas in a projection to a plane perpendicular to the longitudinal axis (L), **characterized in that** an object (4) located in the X-ray capturing area (7) is radiographed by X-ray light of at least two different X-ray sources (8a-8g, 8u-8z) of the X-ray apparatus (6).
